# EUROPEAN PATENT APPLICATION

(11) **EP 1 400 601 A1**
(43) Date of publication of application: **24.03.2004**
(21) Application number: 03255859.5
(22) Date of filing: 18.09.2003
(51) Int. Cl.: C12Q 1/68

(54) **Method for detection of multiple nucleic acid sequence variations**

(30) Priority: 20.09.2002 US 412477 P
(71) Applicant: Bayer Healthcare LLC, Tarrytown, New York 10591-5097 (US)
(72) Inventor: Quinn, John J., Concord California 94521 (US); Warner, Brian, Martinez California 94553 (US); Weare, John, El Sobrante California 94803 (US)
(74) Representative: Duckworth, Timothy John

(57) **Abstract**

A method for detecting the presence or absence of a genetic variation at a polymorphic site in a nucleic acid analyte in a sample is provided. The method comprises a series of steps used to form captured wild type complexes and captured variant complexes that are detected and counted. The method is carried out using first and second differential hybridization probes, first and second capture probes, and first and second solid substrates, each having a detectable signal. The invention also provides for kits for carrying out the assay.

SEQUENCE LISTING

<110> BAYER HEALTHCARE LLC

<120> METHOD FOR DETECTION OF MULTIPLE NUCLEIC ACID SEQUENCE
      VARIATIONS

<130> 1300-0008EP

<140>
<141>

<150> 60/412,477
<151> 2002-09-20

<160> 26

<170> PatentIn Ver. 2.1

<210> 1
<211> 20
<212> DNA
<213> Artificial Sequence

<220>
<223> Description of Artificial Sequence: Primer

<400> 1
tagtggccat cttcctgctc                                              20


<210> 2
<211> 19
<212> DNA
<213> Artificial Sequence

<220>
<223> Description of Artificial Sequence: Primer

<400> 2
tctggtaggg gagcctcag                                               19


<210> 3
<211> 18
<212> DNA
<213> Artificial Sequence

<220>
<223> Description of Artificial Sequence: Primer

<400> 3
cttcggggac gtgttcag                                                18


<210> 4
<211> 20

<212> DNA
<213> Artificial Sequence

<220>
<223> Description of Artificial Sequence: Primer

<400> 4
tcccacggaa atctgtctct                                                    20


<210> 5
<211> 19
<212> DNA
<213> Artificial Sequence

<220>
<223> Description of Artificial Sequence: Primer

<400> 5
cccgttctgt cccgagtat                                                     19


<210> 6
<211> 19
<212> DNA
<213> Artificial Sequence

<220>
<223> Description of Artificial Sequence: Primer

<400> 6
gtttcccaga tgggctcac                                                     19


<210> 7
<211> 20
<212> DNA
<213> Artificial Sequence

<220>
<223> Description of Artificial Sequence: Primer

<400> 7
ccatggtgtc tttgctttcc                                                    20


<210> 8
<211> 20
<212> DNA
<213> Artificial Sequence

<220>
<223> Description of Artificial Sequence: Primer

<400> 8
gtggggtaag caggaatgag                                                    20

<210> 9
<211> 55
<212> DNA
<213> Artificial Sequence

<220>
<223> Description of Artificial Sequence: Probe

<400> 9
gtatgcgcgc tgctatgccg cctggtgggt agccggcgcc tggacgatcc tatct      55


<210> 10
<211> 55
<212> DNA
<213> Artificial Sequence

<220>
<223> Description of Artificial Sequence: Probe

<400> 10
gtatgcgcgc tgctatgccg cctggtgagt agccaaggtg gcaaggaccg tgcct      55


<210> 11
<211> 56
<212> DNA
<213> Artificial Sequence

<220>
<223> Description of Artificial Sequence: Probe

<400> 11
gtatgcgcgc tgctatgccg atctgggtga tggggaggca cgtcggatgc atcgct     56


<210> 12
<211> 56
<212> DNA
<213> Artificial Sequence

<220>
<223> Description of Artificial Sequence: Probe

<400> 12
gtatgcgcgc tgctatgccg atctggatga tggggatcgg caacgcaccc tggtgt     56


<210> 13
<211> 57
<212> DNA
<213> Artificial Sequence

<220>
<223> Description of Artificial Sequence: Probe

<400> 13
gtatgcgcgc tgctatgccg ctatgcgcag gttctgcacg ctggacagag ccaacgt    57

<210> 14
<211> 59
<212> DNA
<213> Artificial Sequence

<220>
<223> Description of Artificial Sequence: Probe

<400> 14
gtatgcgcgc tgctatgccg ctatgcacag gttcgaggat ccacggagcc cttggagtt  59


<210> 15
<211> 57
<212> DNA
<213> Artificial Sequence

<220>
<223> Description of Artificial Sequence: Probe

<400> 15
gtatgcgcgc tgctatgccg gatgggctca ccaagcactc gagtgcgatg taccggt    57


<210> 16
<211> 57
<212> DNA
<213> Artificial Sequence

<220>
<223> Description of Artificial Sequence: Probe

<400> 16
gtatgcgcgc tgctatgccg gatggggtca ccacggccac gctctcagcg aagtgtt    57


<210> 17
<211> 33
<212> DNA
<213> Artificial Sequence

<220>
<223> Description of Artificial Sequence: Probe

<400> 17
cgatggtggt cccggcgcct ggacgatcct atc                              33


<210> 18
<211> 33
<212> DNA
<213> Artificial Sequence

<220>
<223> Description of Artificial Sequence: Probe

<400> 18
cgatgatggt cccaaggtgg caaggaccgt gcc                                33

<210> 19
<211> 18
<212> DNA
<213> Artificial Sequence

<220>
<223> Description of Artificial Sequence: Primer

<400> 19
taactcgtgc tggtggcc                                                 18

<210> 20
<211> 15
<212> DNA
<213> Artificial Sequence

<220>
<223> Description of Artificial Sequence: Primer

<400> 20
aaagcccggc gcaag                                                    15

<210> 21
<211> 35
<212> DNA
<213> Artificial Sequence

<220>
<223> Description of Artificial Sequence: Probe

<400> 21
gggtagtggg tctagaggca cgtcggatgc atcgc                              35

<210> 22
<211> 35
<212> DNA
<213> Artificial Sequence

<220>
<223> Description of Artificial Sequence: Probe

<400> 22
gggtagtagg tctagatcgg caacgcacgc tggtg                              35

<210> 23
<211> 36
<212> DNA
<213> Artificial Sequence

<220>
<223> Description of Artificial Sequence: Probe

<400> 23
cttggacgcg tatctgcacg ctggacagag ccaacg                                36


<210> 24
<211> 38
<212> DNA
<213> Artificial Sequence

<220>
<223> Description of Artificial Sequence: Probe

<400> 24
cttggacacg tatcgaggat ccacggagcc cttggagt                              38


<210> 25
<211> 35
<212> DNA
<213> Artificial Sequence

<220>
<223> Description of Artificial Sequence: Probe

<400> 25
accactcggg taggctcgtg ctcaggcaag gctgc                                 35


<210> 26
<211> 36
<212> DNA
<213> Artificial Sequence

<220>
<223> Description of Artificial Sequence: Probe

<400> 26
accactgggg tagcggccac gctctcagcg aagtgt                                36

## Description

### TECHNICAL FIELD

This invention relates generally to a method for determining the sequence of a nucleic acid target at a polymorphic site. More specifically, the invention relates to a method of determining sequences using direct hybridization of the nucleic acid target. The invention has utility in the fields of diagnostic assays, multiplexing technology, and genetic analysis.

### BACKGROUND

Living organisms possess genetic information in the form of particular sequences of nucleotides. Even within the same species, the sequence of nucleotides that codes for a specific individual can vary. By examining these variations, scientists have been able to correlate specific traits, conditions, or diseases to particular variations in the genetic sequence. Consequently, determination of a genetic sequence at a particular location, commonly referred to as a polymorphic site, can enable the diagnosis of certain genetic diseases, and can reveal a wealth of additional information concerning an individual.

Several techniques for determining the particular sequence at a polymorphic site have been reported in the literature. Specific methods include those based on oligonucleotide ligation and primer extension assays.

Assays based on oligonucleotide ligation use two separate oligonucleotide probes designed to hybridize in a tandem arrangement onto the nucleic acid target, thereby forming an oligonucleotide duplex when perfect complementarity exists between both oligonucleotide probes and the target. That is, one end of each oligonucleotide probe is immediately adjacent to the other when the target and pair of oligonucleotide probes are complementary to each other. Once a sufficient incubation time for hybridization has passed, an enzyme, e.g., a DNA ligase, is added in order to enzymatically ligate, or join, pairs of hybridized oligonucleotide probes adjacent to, although unconnected with, each other on the target. As a consequence, ligation succeeds when both oligonucleotides are complementary to the target, but fails (or is less likely to occur) when exact complementarity is absent. Subsequent heating results in denaturing of the hybridized strands, causing the original oligonucleotide probes to either join to form a single, larger oligonucleotide, or remain as two separate oligonucleotide probes. Detection of the sizes of the resulting larger probes indicates whether there was exact complementariness between the probes and the target. Similar assays based on oligonucleotide ligation are described in U.S. Patent No. 6,025,139 to Yager et al. and Iannone et al. (2000), *Cytometry*, 39:131-140.

Primer extension assays represent another technique often used to identify variations of a single oligonucleotide in a target sequence, commonly referred to as a single nucleotide polymorphism, or "SNP." Primer extension assays have been described in the literature by, for example, Syvänen et al. (1990) *Genomics* 8(4): 684-692. In this technique, a specifically designed primer is hybridized to the target sequence immediately 3' of the single nucleotidic position of interest. A DNA polymerase is added in the presence of an excess of one type of a labeled nucleotide, e.g., a labeled adenosine triphosphate. Detection of a labeled primer, rather than a series of uncoupled labeled nucleotides, allows for the determination of the nucleotide at the position of interest in the target, based on the complementary residue of the labeled oligonucleotide added with DNA polymerase.

While both of the above-identified techniques provide the ability to differentiate between variations in a target sequence, both approaches have significant drawbacks. For example, both steps require the presence of enzymes, e.g., DNA ligase for oligonucleotide ligase assays, and DNA polymerase for primer extension assays. These enzymes increase the overall cost of the assay and require additional steps such as washing, which tend to further complicate the assays. Additionally, these approaches are time consuming and do not lend themselves to multiplexed formats wherein clusters or separate polymorphisms are simultaneously detected.

A third technique based on competitive hybridization has been suggested to overcome at least some of these shortcomings. The competitive hybridization approach uses two oligonucleotide probes, one complementary to the variant of interest and the other complementary to the natural or "wild type" sequence. Based on the hybridization results, it is possible to determine which sequence, i.e., the variant or wild type, is contained in the target. A specific example of this technique is described in U.S. Patent No. 6,187,538 to Eastman et al. Other than using a polymerase to amplify the target, no additional enzymes are required.

Competitive hybridization assays have also been adapted into a multiplex format. U.S. Patent No. 6,057,107 to Fulton describes a method for flow cytometric determination of DNA sequences based on oligonucleotide probe competition. In the described method, a specialized bead set is used wherein uniquely labeled beads are directly coupled to an oligonucleotide probe designed to interrogate the polymorphic region of interest. Initially, the oligonucleotide probes of the bead set are hybridized to a complementary labeled oligonucleotide. When the polymorphic region in the target is also complementary to the probe, competitive displacement of the labeled oligonucleotide results, thereby effecting a means to differentiate the sequence at the polymorphic site. The complexes "captured" by the uniquely labeled beads are passed through a flow cytometer for detection of the label from the originally hybridized complementary labeled oligonucleotide (if present) and determination of the bead type. Sequence differentiation is based on whether the label from the originally hybridized complementary labeled probe is detected from the captured complex: a decrease in the expected label signal (as determined from a control, for example) indicates that the sequence is the same as the originally labeled probe, since the unlabeled target displaced the labeled oligonucleotide, while no change in label signal indicates a different sequence, as no displacement occurred.

Assays based on competitive hybridization as described above, however, suffer from drawbacks as well. For example, the bead sets are unique for each specific variation to be identified, thereby requiring vast amounts of preparation in coupling unique probes to each bead. Thus, while multiplexing is nonetheless possible with this approach, such competitive hybridization assays are not easily adaptable for sequence determination at other sites. Thus, there remains a need to provide assays that determine nucleic acid sequences easily and in a manner that can easily be adapted for detecting other polymorphic sites.

### SUMMARY OF THE INVENTION

It is thus a primary object of the present invention to address the above-mentioned need in the art by providing a method for detecting the presence or absence of a genetic variation at a polymorphic site in a nucleic acid analyte in a sample, comprising the steps of: (a) preparing labeled amplicons from the nucleic acid analyte contained in the sample; (b) contacting, under hybridization conditions, the labeled amplicons with a plurality of first and second differential hybridization probes to form wild type and variant complexes; (c) contacting, under hybridization conditions, any wild type complexes and variant type complexes formed in step (b) with a plurality of first and second capture probes to form captured wild type and variant complexes; (d) detecting and counting any captured wild type complexes and captured variant complexes formed in step (c); and (e) determining the presence or absence of the genetic variation by comparing the relative amounts of the captured wild type and captured variant complexes detected in step (d), wherein a greater amount of captured wild type complexes is indicative of the absence of the genetic variation, and a greater amount of captured variant complexes is indicative of the presence of the genetic variation.

It is still another object of the invention to provide such a method wherein the captured wild type complexes and variant type complexes are detected through detectable signals provided by solid substrates.

Another object of the invention is to provide such a method wherein the solid substrates are beads.

Still another object of the invention is to provide such a method wherein each detectable signal is based on a ratio of two different fluorescent dyes.

In still a further object of the invention to provide an assay kit for carrying out the inventive method.

Additional objects, advantages, and novel features of the invention will be set forth in the description that follows, and in part, will become apparent to those skilled in the art upon examination of the following, and may be learned by practice of the invention.

In a first embodiment, the invention provides a method for detecting the presence or absence of a genetic variation at a polymorphic site in a nucleic acid analyte in a sample. The method includes the step of preparing labeled amplicons from the nucleic acid analyte contained in the sample. Generally, although not necessarily, the nucleic acid amplification step is carried out using conventional polymerase chain reaction (PCR) techniques, typically with the aid of a labeled primer such as a biotinylated primer.

Next, the labeled amplicons are contacted under hybridization conditions with a plurality of first and second differential hybridization probes. Each probe in the plurality of first differential hybridization probes is comprised of a first capture sequence portion and a region that is complementary to the polymorphic site corresponding to a wild type sequence. Each probe in the plurality of second differential hybridization probes is comprised of a second capture portion that is different from the first capture portion. Furthermore, each second differential hybridization probe also comprises a region that is complementary to the polymorphic site corresponding to a variation sequence. Thus, when the differential hybridization probes are contacted to the labeled amplicons under hybridizing conditions, wild type complexes are formed between the first hybridization probes and labeled amplicons having the polymorphic site corresponding to the wild type sequence. Similarly, variant complexes are formed between the second hybridization probes and labeled amplicons having the polymorphic site corresponding to the variant sequence.

Once formed, the wild type and/or variant complexes are contacted under hybridization conditions with a plurality of first and second capture probes. The first capture probes are each comprised of a region that is complementary to the first capture sequence portion. In addition, each first capture probe is attached to a single solid substrate having a first detectable signal. The second capture probes, in turn, are each comprised of a region that is complementary to the second capture sequence portion. Similarly, each second capture probe is attached to a single solid substrate having a second detectable signal. Thus, captured wild type complexes are formed between any wild type complexes and first capture probes, and captured variant complexes are formed between variant complexes and second capture probes. All the solid substrates used in the method are preferably beads, although other shapes may be used.

The captured complexes, wild type and/or variant, are then detected and counted. By comparing the relative amounts of the captured wild type and captured variant complexes, one can determine whether the nucleic acid sample is positive or negative for the genetic variation being sought. Specifically, a greater amount of captured wild type complexes is indicative of the absence of the genetic variation, while a greater amount of captured variant complexes is indicative of the presence of the variation. A heterozygous condition may exist in the sample when an equal or substantially equal amount of both types of complexes are detected.

In a second embodiment, an assay kit is provided, wherein the kit comprises: (a) a plurality of first differential hybridization probes each comprised of a first capture portion and a region that is complementary to the polymorphic site corresponding to a wild type sequence; (b) a plurality of second differential hybridization probes each comprised of a second capture portion different from the first capture portion and a region that is complementary to the polymorphic site corresponding to a variation sequence; (c) a plurality of first solid substrates each (i) comprised of an attached first capture probe that is complementary to the first capture portion and (ii) having a first detectable signal; and (d) a plurality of second solid substrates each (i) comprised of an attached second capture probe that is complementary to the second capture portion and (ii) having a second detectable signal. The assay kit optionally includes additional components, such as a polymerase for amplifying nucleic acids; primers (preferably labeled) for carrying out PCR; and instructions for carrying out the assay.

The present invention offers many advantages over prior techniques. For example, the present method provides the ability to detect all types of genetic variations, including single nucleotide polymorphisms as well as individual or clusters of polymorphisms. In addition, the inventive method does not require the use of enzymes once amplification of the nucleic acid target takes place. Furthermore, the present method can be carried out simultaneously with different probes in a "one-pot" approach, thereby allowing for convenient multiplexing. Moreover, the method and components used therein are well suited for large-scale, commercial applications. For example, uniquely fluorescent beads containing known capture probe sequences attached thereto can be adapted for the detection of any number of genetic assays, since only the differential hybridization probes need to be adjusted without attaching a unique probe to the bead.

### BRIEF DESCRIPTION OF THE DRAWINGS

FIG. 1 illustrates the time allotted for each step of the SNP assay.

FIG. 2 shows the design of the primers, probes, and capture sequences of the CYP2D6 SNP Assay method (SEQ ID NOS: 17-22, 5-6, 23-24, 7-8 and 25-26, respectively, in order of appearance).

FIG. 3 schematically illustrates the primers and probes used to detect an SNP in multiplex mode, and using Luminex™ microspheres.

FIG. 4 is a graph bar demonstrating the cytochrome P450 genotyping results of a multiplex SNP assay conducted on four individual patient samples.

### DETAILED DESCRIPTION OF THE INVENTION

Before describing the present invention in detail, it is to be understood that unless otherwise indicated this invention is not limited to specific sources of DNA, specific sequences, or specific assay formats, as such may vary. It is also to be understood that the terminology used herein is for the purpose of describing particular embodiments only, and is not intended to be limiting.

It must be noted that, as used in this specification and the appended claims, the singular forms "a," "an," and "the" include plural referents unless the context clearly dictates otherwise.

In describing and claiming the present invention, the following terminology will be used in accordance with the definitions set out below.

It will be appreciated that, as used herein, the terms "nucleoside" and "nucleotide" refer to nucleosides and nucleotides containing not only the conventional purine and pyrimidine bases, i.e., adenine (A), thymine (T), cytosine (C), guanine (G), and uracil (U), but also modified nucleosides and nucleotides. Such modifications include, but are not limited to, methylation or acylation of a purine or pyrimidine moiety, substitution of a different heterocyclic ring structure for a pyrimidine ring or for one or both rings in the purine ring system, and protection of one or more functionalities, e.g., using a protecting group such as acetyl, difluoroacetyl, trifluoroacetyl, isobutyryl, benzoyl, and the like. Modified nucleosides and nucleotides also include modifications on the sugar moiety, e.g., wherein one or more of the hydroxyl groups are replaced with halide and/or hydrocarbyl substituents (typically aliphatic groups, in the latter case), or are functionalized as ethers, amines, or the like. Common analogs include, but are not limited to, 1-methyladenine, 2-methyladenine, N⁶-methyladenine, N⁶-isopentyl-adenine, 2-methylthio-N⁶-isopentyladenine, N,N-dimethyladenine, 8-bromoadenine, 2-thiocytosine, 3-methylcytosine, 5-methylcytosine, 5-ethylcytosine, 4-acetylcytosine, 1-methylguanine, 2-methylguanine, 7-methylguanine, 2,2-dimethylguanine, 8-bromo-guanine, 8-chloroguanine, 8-aminoguanine, 8-methylguanine, 8-thioguanine, 5-fluoro-uracil, 5-bromouracil, 5-chlorouracil, 5-iodouracil, 5-ethyluracil, 5-propyluracil, 5-methoxyuracil, 5-hydroxymethyluracil, 5-(carboxyhydroxymethyl)uracil, 5-(methyl-aminomethyl)uracil, 5-(carboxymethylaminomethyl)-uracil, 2-thiouracil, 5-methyl-2-thiouracil, 5-(2-bromovinyl)uracil, uracil-5-oxyacetic acid, uracil-5-oxyacetic acid methyl ester, pseudouracil, 1-methylpseudouracil, queosine, inosine, 1-methylinosine, hypoxanthine, xanthine, 2-aminopurine, 6-hydroxyaminopurine, 6-thiopurine, and 2,6-diaminopurine. Iso-guanine and iso-cytosine may be incorporated into oligonucleotides to lower potential cross reactivity between sequences when hybridization is not desired.

As used herein, the term "oligonucleotide" encompasses polydeoxyribonucleotides (containing 2-deoxy-D-ribose), polyribonucleotides (containing D-ribose), any other type of polynucleotide that is an N-glycoside of a purine or pyrimidine base, and other polymers containing nonnucleotidic backbones (e.g., protein nucleic acids and synthetic sequence-specific nucleic acid polymers commercially available from the Anti-Gene Development Group, Corvallis, Oregon, as Neugene™ polymers) or nonstandard linkages, providing that the polymers contain nucleobases in a configuration that allows for base pairing and base stacking, such as is found in DNA and RNA. Thus, "oligonucleotides" herein include double- and single-stranded DNA, as well as double- and single-stranded RNA and DNA:RNA hybrids, and also include known types of modified oligonucleotides, such as, for example, oligonucleotides wherein one or more of the naturally occurring nucleotides is substituted with an analog; oligonucleotides containing internucleotide modifications such as, for example, those with uncharged linkages (e.g., methyl phosphonates, phosphotriesters, phosphoramidates, carbamates, etc.), negatively charged linkages (e.g., phosphorothioates, phosphorodithioates, etc.), and positively charged linkages (e.g., aminoalkylphosphoramidates, aminoalkylphosphotriesters), those containing pendant moieties, such as, for example, proteins (including nucleases, toxins, antibodies, signal peptides, poly-L-lysine, etc.), those with intercalators (e.g., acridine, psoralen, etc.), those containing chelators (e.g., metals, radioactive metals, boron, oxidative metals, etc.), and those containing alkylators. There is no intended distinction in length between the terms "polynucleotide" and "oligonucleotide," and these terms will be used interchangeably. These terms refer only to the primary structure of the molecule. As used herein the symbols for nucleotides and polynucleotides are according to the IUPAC-IUB Commission of Biochemical Nomenclature recommendations (*Biochemistry* 9:4022, 1970).

By "PCR" is meant herein the polymerase chain reaction (PCR) technique, disclosed by Mullis in U.S. Patent Nos. 4,683,195 (Mullis et al.) and 4,683,202, incorporated herein by reference. In the PCR technique, short oligonucleotide primers are prepared that match opposite ends of a desired sequence. The sequence between the primers need not be known. A sample of DNA (or RNA) is extracted and denatured (preferably by heat). Oligonucleotide primers are then added in molar excess, along with dNTPs and a polymerase (preferably Taq polymerase, which is stable to heat). The DNA is replicated, then again denatured. This results in two "long products," which begin with the respective primers, and the two original strands (per duplex DNA molecule). The reaction mixture is then returned to polymerizing conditions (e.g., by lowering the temperature, inactivating a denaturing agent, or adding more polymerase), and a second cycle is initiated. The second cycle provides the two original strands, the two long products from cycle 1, two new long products (replicated from the original strands), and two "short products" replicated from the long products. The short products have the sequence of the target sequence (sense or antisense) with a primer at each end. On each additional cycle, an additional two long products are produced, and a number of short products equal to the number of long and short products remaining at the end of the previous cycle. Thus, the number of short products grows exponentially with each cycle. This amplification of a specific analyte sequence allows the detection of extremely small quantities of DNA.

The term "3SR" as used herein refers to a method of target nucleic acid amplification also known as the "self-sustained sequence replication" system as described in European Patent Publication No. 373,960 (published Jun. 20, 1990).

The term "LCR" as used herein refers to a method of target nucleic acid amplification also known as the "ligase chain reaction" as described by Barany (1991), *Proc. Nat. Acad. Sci*. 88:189-193.

As used herein, the term "probe" refers to a structure comprised of a polynucleotide that forms a hybrid structure with a target sequence contained in a molecule (a "target molecule") in a sample undergoing analysis, due to complementarity of at least one sequence in the probe with the target sequence. The nucleotides of any particular probe may be deoxyribonucleotides, ribonucleotides, and/or synthetic nucleotide analogs. The term "primer" refers to a molecule that comprises an oligonucleotide, whether produced naturally as in a purified restriction digest or produced synthetically, which is capable of acting as a point of initiation of synthesis when placed under conditions in which synthesis of a primer extension product that is complementary to a nucleic acid strand is induced, i.e., in the presence of appropriate nucleotides and an agent for polymerization such as a DNA polymerase in an appropriate buffer and at a suitable temperature. Preferred primers for use herein are the dual-purpose primers described in European Patent Application No. to Quinn et al. for "Dual-Purpose Primers and Probes for Providing Enhanced Hybridization Assays by Disruption of Secondary Structure Formation," filed on even date herewith.

It will be appreciated that the binding sequences need not have perfect complementarity to provide stable hybrids. In many situations, stable hybrids will form where fewer than about 10% of the bases are mismatches, ignoring loops of four or more nucleotides. However, the high sensitivity of this assay will usually require that the probes that hybridize to the first and second variants of the target nucleotide sequence and the control nucleotide sequence have 100% homology to their targets. Accordingly, as used herein the term "complementary" intends to refer to an oligonucleotide that forms a stable duplex with its "complement" under assay conditions, generally where there is about 100% homology, while the term "substantially complementary" intends to refer to an oligonucleotide that forms a stable duplex with its "complement" under assay conditions, generally where there is about 90% or greater homology.

The term "hybridizing conditions" is intended to mean those conditions of time, temperature, and pH, and the necessary amounts and concentrations of reactants and reagents, sufficient to allow at least a portion of complementary sequences to anneal with each other. As is well known in the art, the time, temperature, and pH conditions required to accomplish hybridization depend on the size of the oligonucleotide probe to be hybridized, the degree of complementarity between the oligonucleotide probe and the target, and the presence of other materials in the hybridization reaction admixture. The actual conditions necessary for each hybridization step are well known in the art or can be determined without undue experimentation.

Typical hybridizing conditions include the use of solutions buffered to a pH from about 7 to about 8.5 and temperatures of from about 30°C to about 60°C, preferably from about 37°C to about 55°C for a time period of from about one second to about one day, preferably from about 15 minutes to about 16 hours, and most preferably from about 15 minutes to about three hours.

"Hybridization conditions" also include an effective buffer. Any buffer that is compatible, i.e., chemically inert, with respect to the probes and other components, yet still allows for hybridization between complementary base pairs, can be used. One particularly preferred buffer comprises 3X SSC, 50% formamide, 10% dextran sulfate (MW 500,000), 0.2% casein, 10 µg/mL poly A, and 100 µg/mL denatured salmon sperm DNA, wherein 1X SSC is 0.15 M sodium chloride and 0.015 M sodium citrate. Another particularly preferred buffer comprises 5X SSC, 0.1 to 0.3% sodium dodecyl sulfate, 10% dextran sulfate, 1 mM ZnCl₂, and 10 mM MgCl₂, wherein 1X SSC is as defined above. Other suitable buffers are known to those of ordinary skill in the art.

"Patient" as used herein refers to an organism, preferably mammalian, from which the nucleic acid analyte is obtained. Preferred patients are mammalian organisms, e.g., humans.

As used herein, the term "sample" refers to a fluid or tissue obtained from an organism (e.g., a mammalian organism such as a human) that contains the nucleic acid analyte to be characterized. Such samples are known in the art and include, without limitation: blood; plasma; serum; spinal fluid; lymph fluid; cell lysates; semen; secretions of the skin or respiratory, intestinal, or genitourinary tracts; tears; saliva; milk; and white blood cells.

The term "attached" refers to coupling by covalent or non-covalent interactions (e.g., hydrophobic interactions, hydrogen bonds, etc.). Covalent bonds include, for example, ester, ether, phosphoester, amide, imide, carbon-sulphur bonds, carbon-phosphorous bonds, and the like. Methods for attaching oligonucleotides to substrates are known in the art and include, for example, blotting of the oligonucleotide onto the substrate.

The term "substrate" refers to any solid or semi-solid surface to which a desired oligonucleotide may be anchored. Suitable substrates include any material that can immobilize an oligonucleotide and encompass, for example, glass, nitrocellulose, plastics including polyvinyl chloride (e.g., in sheets or microtiter wells), polystyrene latex (e.g., in beads or microtiter plates), polyvinylidine fluoride (e.g., in microtiter plates), polystyrene (e.g., in beads), metal, polymer gels, and the like.

"Optional" or "optionally" means that the subsequently described event or circumstance may or may not occur, and that the description includes instances where the said event or circumstance occurs as well as instances where it does not. For example, reference to an "optional component" in an assay kit means that such a component may or may not be present in the assay kit, and the description includes assay kits wherein the component is present and assay kits wherein the component is not present.

Although any similar or equivalent methods and materials may be employed in the practice or testing of the present invention, preferred methods and materials are now described.

The method of this invention comprises a means for detecting the presence or absence of a genetic variation, e.g., mutation, in a nucleic acid analyte obtained from a sample. Because the genetic variation results in an altered sequence (e.g., a missing, changed, or added nucleotide) the sequence itself is considered "polymorphic," and the site at which the alteration occurs is referred to as a "polymorphic site." Thus, the nucleic acid analyte of interest is a portion of genetic material that comprises or is suspected to comprise a polymorphic site.

The method includes the preparation of labeled amplicons from the nucleic acid analyte contained in the sample. The actual steps of preparing labeled amplicons are well known to those of ordinary skill in the art. For example, the nucleic acid analyte of interest contained in the sample is often located within a cell or virus. The nucleic acid analyte can be DNA, RNA, or some other nucleic acid-containing polymer, which can either be in single-stranded or double-stranded form.

Cells containing the nucleic acid analyte of interest can be obtained from biological tissue or fluid samples using conventional techniques such as, for example, needle biopsy or swabbing. The cells so obtained may contain genetic material of the host, or they may contain non-host genetic material. Thus, for example, samples obtained from a human individual may contain human genetic material and/or the genetic material of an infectious pathogen such as a bacterium, virus, or fungus.

Once obtained, the nucleic acid analyte is then amplified, i.e., copied, using conventional techniques. Although any known amplification method can be used, preferred methods include PCR, 3SR, and LCR, with PCR being most preferred.

As outlined above, PCR amplification is carried out in a mixture comprising the nucleic acid analyte, a polymerase (e.g., Taq polymerase), oligonucleotide primers, an excess of the four oligonucleotide (dNTP) monomers, and water that contains a buffer suitable for carrying out PCR (including conventional buffers comprising Tris-HCl, KCl, and MgCl₂). The mixture is then exposed to a series of replication cycles based on temperature. For example, the mixture is heated to about 94-96°C for several minutes during which time any double-stranded DNA is denatured into single-stranded DNA. Next, the temperature of the mixture is lowered to about 50-65°C, during which time the oligonucleotide primers hybridize via hydrogen bonds to complementary sequences. Finally, the temperature of the mixture is increased to about 72°C, during which time the polymerase binds and extends a complementary strand from each primer. Since the sequence being amplified doubles after each sample, a theoretical amplification of one billion can be attained, thereby providing ample nucleic acid analyte for the present method.

Any primers suitable for amplifying the nucleic acid analyte may be used in the PCR process. Primers are relatively short oligonucleotides (e.g., 10 - 30 bases in length) that are complementary to a portion of the nucleic acid to be amplified. The primers are annealed to the denatured nucleic acid and provide an initiation site for elongation of the new DNA molecule. Some primers can be specific to a particular nucleic acid sequence, while others are universal in nature, such that they anneal at locations found throughout the genome of a single organism or in the genomes of several organisms. Suitable PCR primers are prepared by means known to those of ordinary skill in the art, for example, by cloning and performing restriction of appropriate sequences, or by direct chemical synthesis. For example, one may employ the phosphotriester method described by S. A. Narang et al. (1979) *Meth. Enzymol*. 68:90, and U.S. Patent No. 4,356,270 to Itakura. Primers are also available from Sigma-Aldrich, Co. (St. Louis, Missouri) and other commercial suppliers. Other primers may also be used, such as those designed to hybridize to other portions of the nucleic acid analyte in order to avoid undesired secondary structure.

For use in the present method, the amplified nucleic acid analyte, referred to herein as the "amplicon," must be labeled. In a first technique, labeled primers are used during the amplification step, thereby resulting in labeled amplicons as the labeled primers are incorporated into the amplicons. Labeled primers are available from commercial suppliers such as CPG, Inc. (Lincoln Park, New Jersey), or they can be prepared via conjugating a label to an unlabeled primer. Labeling oligonucleotides such as primers can be carried out using conventional coupling procedures. Specific coupling procedures, however, will vary depending on the reactive group or groups present on the label and/or on the oligonucleotide.

For example, nick translation procedures known to those of skill in the art are available for substituting an unlabeled nucleotide with a labeled nucleotide through the use of DNase I and other enzymes, thereby providing a labeled amplicon. Another method for labeling includes the addition of a labeled deoxynucleotide terminal deoxynucleotidyl transferase (TdT, available from commercial suppliers such as PanVera Corp., Madison, Wisconsin), a DNA polymerase that can catalyze the addition of labeled deoxynucleotides to the 3'-end of DNA fragments.

A second technique for preparing labeled amplicons involves a separate labeling step wherein unlabeled amplicons are subsequently coupled to a label. The techniques described above with respect to labeling primers can also be used to attach labels to unlabeled amplicons.

Any type of label can be attached to the amplicon. Preferred labels include those moieties detectable by spectroscopic, photochemical, biochemical, immunochemical, or chemical means. Such labels include, without limitation, fluorescers, chemiluminescers, dyes, biotin, haptens, enzymes, enzyme substrates, enzyme cofactors, enzyme inhibitors, enzyme subunits, metal ions, electron-dense reagents, and radioactive isotopes (e.g., ³²P). The label moiety can be directly or indirectly attached to the amplicon. Preferably, the label should be selected to withstand denaturing conditions if it is to be attached directly to the primer. It is preferred, although not necessary, that the label be biotin, which can be detected via binding with streptavidin coupled to a fluorescer, e.g., a streptavidin-phycoerythrin conjugate.

Once prepared, the labeled amplicons are contacted with a plurality of first and second differential hybridization probes under conditions that permit hybridization. The first differential hybridization probe comprises two binding regions: a first capture sequence portion and a region that is complementary to the polymorphic site corresponding to a wild type sequence. Optionally, the first hybridization probe further comprises a spacer portion. Thus, wild type complexes are formed between the first hybridization probe and the nucleic acid analyte when the polymorphic site of nucleic acid analyte is in the "natural" or wild type form.

Similarly, second differential hybridization probes comprising two binding regions and an optional spacer portion are also contacted with the labeled amplicons under hybridization conditions. The two binding regions, however, differ from the first differential hybridization probes in that the second hybridization probes each comprise a second capture sequence portion and a region that is complementary to the polymorphic site corresponding to a variant sequence. Consequently, any of the labeled amplicons made from a nucleic acid analyte having the variant sequence at the polymorphic site will hybridize to the corresponding complementary region of the second differential hybridization probe, thereby forming variant complexes.

The optional spacer portion in the hybridization probes can be located at any part of the probe. It is preferred, however, that the spacer portion be located between the capture sequence portion and the region that is complementary to the polymorphic site. The spacer can comprise a series of nucleotides specifically designed not to hybridize or interfere with the hybridization events necessary for carrying out the present method, i.e., designed not to interfere with binding to other probes. In addition, nonnucleotidic spacers may be used. Methods suitable for preparing the optional spacer are known from the literature. The preparation of polyethylene glycol spacers is described, for example, by Kern et al. (1979) *Makromol. Chem*. 150:2539. Other spacers can be prepared in a similar manner.

The amount added of each of the first and second hybridization probes and the time required for hybridization can be determined experimentally, but it is preferred that a molar excess of each probe type be used. Generally, however, each of the first and second hybridization probes are added in an amount of from about 0.01 pmoles to about 100 pmoles, with a hybridization incubation period of from about one minute to about one day. It is preferred, however, that each of the first and second hybridization probes be added in an amount of from about 0.1 pmoles to about 10 pmoles for each type of probe, and allowed to incubate for a period of time lasting from about five minutes to about thirty minutes.

Once a sufficient incubation period has passed, variant complexes and/or wild type complexes will have formed (as discussed above), depending upon the polymorphic form of the nucleic acid analyte. These complexes, if any, are then "captured" using capture probes coupled to a solid substrate or a plurality of solid substrates. Each wild type complex is captured by a first capture probe comprised of a region that is complementary to the first capture sequence portion of the first differential hybridization probe. The first capture probe is coupled to a solid substrate. A plurality of first capture probes is used in order to capture substantially all of the wild type complexes. The variant complexes are captured in a similar way, recognizing that a plurality of second capture probes, each comprised of a region that is complementary to the second capture sequence portion and each attached to a plurality of second solid substrates, is used to capture the variant sequences. The preferred molar amounts and times used for capturing the complexes are the same as those disclosed above for forming the complexes. The complexes so captured through this step are referred to as "captured wild type complexes" and "captured variant complexes."

Detection and counting of the captured wild type complexes and captured variant complexes can take place using any conventional method. As the captured complexes comprise a label (from the labeled amplicon) and a solid substrate (from the capture probe) having a detectable signal, detection generally proceeds by detecting both the label and the signal.

The labeling can be accomplished by any art-known means and is dependent upon the nature of the label. For fluorescers, a large number of fluorometers are available. For chemiluminescers, luminometers or films are available. With enzymes, a fluorescent, chemiluminescent, or colored product can be provided and determined fluorometrically, luminometrically, spectrophotometrically, or visually (preferably with the aid of a microscope). For example, a biotinylated label is detected by adding a streptavidin-phycoerythrin conjugate, followed by detection of phycoerythrin-induced fluorescence.

Each signal provided by the solid substrate is detected by any conventional means. For example, the signal can be based on the shape of the substrate, wherein the first detectable signal and second detectable signal are differentiated by different shapes of the corresponding solid substrate. It is preferred, however, that each set of solid substrates, e.g., the plurality of first solid substrates, generates a signal unique to the plurality. Thus, for example, each of the first solid substrates has a first detectable signal and each of the second solid substrates has a second detectable signal. Again, the first and second detectable signals can be provided by moieties selected from the groups consisting of fluorescers, chemiluminescers, dyes, biotin, haptens, enzymes, enzyme substrates, enzyme cofactors, enzyme inhibitors, enzyme subunits, metal ions, electron-dense reagents, and radioactive isotopes.

It is most preferred, however, that the solid substrates be beads, wherein a detectable signal is provided by one or more fluorescent dyes. Examples of suitably dyed substrates, e.g., beads, are described in U.S. Patent No. 6,268,222 to Chandler et al. As described therein, the solid substrates comprise a combination of fluorescent or colored dyes. Preferred dyes include cyanine dyes that are characterized by having emission wavelengths of between 550 nm to 900 nm. Some cyanine dyes have a blue to blue-green fluorescence, while others have green to yellow-green fluorescence. Still other cyanine dyes have red or even infrared fluorescence. The cyanine dye, or any other conventional dye, can be covalently attached to the substrate or adsorbed, e.g., "stained," thereon. In addition, the substrate can have attached thereto one or more populations of fluorescently stained nanoparticles, wherein all nanoparticles in a given population have the same dye concentration. By varying the quantity and ratio of different dyes specific to different populations of nanoparticles associated with the substrate, it is possible to establish and distinguish a large number of discreet populations of substrates with unique emission spectra. Such uniquely labeled substrates are particularly useful for multiplex analysis of sequences, and can be conveniently detected and analyzed using flow cytometry. Such beads are also available from commercial suppliers such as, for example, Luminex Corporation (Austin, Texas).

Optimally, a flow cytometer linked with one or more detecting means is used for detecting the complexes, although other means for detecting and counting the captured complexes can also be used, depending on the type of label and signal. The complexes in the hybridization solution are passed through the flow cytometer, thereby allowing the detection of each complex. Preferably, the flow cytometer is linked with a first detecting means for detecting the label (of the labeled amplicon) as well as a second detecting means for detecting the signal associated with the solid substrate. Suitable equipment and methods for detecting the labels and signals using flow cytometry, and having the ability to perform multiplexing analysis, are described in U.S. Patent Nos. 5,981,180 to Chandler et al., and 6,046,807 and 6,139,800 to Chandler. Commercially available systems are also available from Luminex Corp. (Austin, Texas) and include, for example, the Luminex™ 100 machine.

Substrates having a diameter of less than one millimeter can be used in flow cytometers, although other sized particles can be used as well. It is preferred, however, that substrates that are spherical in shape, e.g., beads, be used. Such beads have a size in the range of from about 0.1 to 1,000 µm, preferably 1 to 100 µm, more preferably 2 to 50 µm, still more preferably 3 to 25 µm, with beads having a diameter of from about 6 to 12 µm being most preferred. Beads of this size are suited for use in flow cytometers, thereby providing a facile means for detecting and counting the complexes.

The solid substrates are preferably, although not necessarily, made of a polymeric material such as polystyrene. Other usable polymeric materials include brominated polystyrene, polyacrylic acid, polyacrylonitrile, polyamide, polyacrylamide, polyacrolein, polybutadiene, polycaprolactone, polycarbonate, polyester, polyethylene, polyethylene terephthalate, polydimethylsiloxane, polyisoprene, polyurethane, polyvinyl acetate, polyvinylchloride, polyvinylpyridine, polyvinylbenzylchloride, polyvinyltoluene, polyvinylidene chloride, polydivinylbenzene, polymethylmethacrylate, polylactide, polyglycolide, poly(lactide-co-glycolide), polyanhydride, polyorthoester, polyphosphazene, polyphosophaze, polysulfone, and combinations thereof. These polymers may also incorporate a magnetic or magnetically responsive metal oxide selected from the group consisting of superparamagnetic, paramagnetic, ferrimagnetic, antiferromagnetic, and ferromagnetic metal oxides.

Once all of the captured wild type complexes and captured variant complexes have been detected and counted, the presence or absence of the genetic variation can be determined. Specifically, a greater amount of captured wild type complexes is indicative of the absence of the genetic variation, whereas a greater amount of captured variant complexes is indicative of the presence of the variation. Relative comparisons such as these are sufficient to determine the sequence at a known polymorphic site. Additional techniques can be used, however, to obtain even more information.

For example, ratios can be developed to determine whether the sample contained a mixture of both the wild type and variant sequences. The results can be expressed as the ratio of the log net signals, wherein cutoffs can be used to identify what sequence or combination of sequences is contained in the sample. An example of such a ratio is provided in Table 1.

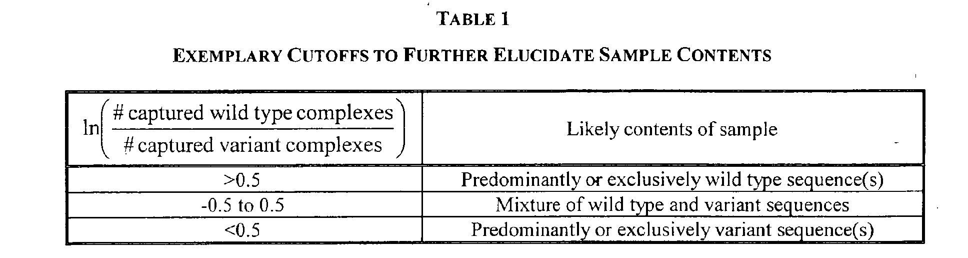

As provided in Table 1, results can be categorized in order to establish whether the sample predominantly or exclusively contains a single sequence type. As also shown in Table 1, results can be obtained wherein a mixture of two types of sequences is contained in a sample, e.g., when the sample is taken from an organism possessing the heterozygous condition.

The invention also provides assay kits for carrying out the method described herein. The assay kits comprise in a packaged combination: (a) a plurality of first differential hybridization probes each comprised of a first capture sequence portion and a region that is complementary to the polymorphic site corresponding to a wild type sequence; (b) a plurality of second differential hybridization probes each comprised of a second capture sequence portion different from the first capture sequence portion and a region that is complementary to the polymorphic site corresponding to a variation sequence; (c) a plurality of first solid substrates each (i) comprised of an attached first capture probe complementary to the first capture region and (ii) having a first detectable signal; and (d) a plurality of second solid substrates each (i) comprised of an attached second capture probe complementary to the second capture region and (ii) having a second detectable signal.

The kit may also contain a polymerase such as a DNA polymerase, Taq polymerase, or similar polymerase for creating amplicons. Primers, preferably labeled, for carrying out PCR may also be included in the assay kits. When labeled, the primers are preferably biotinylated primers. Hybridization buffers, enzyme substrates, negative controls, positive controls, and written instructions for carrying out the method described herein may also be conveniently included in the assay kit.

Methods for preparing the oligonucleotides included with the kits and used in the methods described herein are known to those of ordinary skill in the art. Thus, conventional techniques are used to prepare the necessary primers, first hybridization probes, second hybridization probes, first capture probes, second capture probes, and so forth. For example, the oligonucleotides can be prepared by oligonucleotide synthesis or by cloning, with the former preferred. Direct synthetic methods, for example, typically involve sequential addition of 3'-blocked and 5'-blocked nucleotide monomers to the terminal 5'-hydroxyl group of a growing oligonucleotide chain, wherein each addition is effected by nucleophilic attack of the terminal 5'-hydroxyl group of the growing chain on the 3'-position of the added monomer, which is typically a phosphorus derivative such as a phosphotriester, phosphoramidite, or the like.

The actual sequences of the oligonucleotides are determined using standard techniques known in the art. For each of the differential hybridization probes, it is first necessary to determine the sequence for each polymorphic site, i.e., the wild type and variant sequences. The wild type and variant sequences can be determined manually by first obtaining a sample of the nucleic acid analyte containing the polymorphic site of interest, and then sequencing the nucleic acid analyte using a commercial sequencer such as the Global IR² System (LI-COR, Inc., Lincoln, Nebraska). In addition, the sequence of many nucleic analytes can be determined by referring to the relevant texts and databases providing wild type and variant sequences, such as the GenBank database (Bethesda, Maryland). Once a sequence has been determined, the corresponding complementary sequence is included in the appropriate differential hybridization probe. That is, a region complementary to the polymorphic site corresponding to the wild type sequence is included in the first differential hybridization probe, and a region that is complementary to the variant sequence is included in the second differential hybridization probe.

The sequences used for capturing, i.e., the sequences of the capture portions and the regions on the capture probes complementary thereto, can be directly attached to the solid substrate, or may be spaced therefrom by a spacer. The same types of spacers discussed above with respect to the differential hybridization probes can be used between the solid substrate and the capture sequence. For the capture regions, natural nucleotides can be substituted with non-natural nucleotides such as iso-C and iso- G, and the sequences designed to hybridize to the capture regions can have the corresponding complementary non-natural nucleotides to enhance specificity and reduce background of the capture hybridization step. Methods for using the non-natural nucleotides to reduce nonspecific hybridization are described in U.S. Patent No. 6,232,462 to Collins et al.

Those regions of the oligonucleotides that are intended to be involved with binding (and thus are complementary to another sequence of oligonucleotides, either a probe or analyte) will each be at least 15 nucleotides, usually at least 20 nucleotides, and not more than about 100 nucleotides. Typically, the binding sequences will be approximately 25 nucleotides in length.

The methods described herein have a vast number of uses. For example, the method provides the ability to detect nucleic acid variants, e.g., SNPs, and other useful genetic or epigenomic data such a genetic subtype. In addition, the method can be easily adapted into a multiplex format when the appropriate probes and substrates are used. That is, multiplexed approaches based on the described method will include a different set of first and second hybridization probes, first and second capture probes, and substrates providing a first and second detectable signal for each polymorphic site of interest. Further, the present invention can simultaneously detect clusters of polymorphisms when the appropriate probes and substrates are used. In addition, once the genotype of a particular nucleic acid target is known, it can often be possible to predict the corresponding phenotype based on genetic principles known to those of ordinary skill in the art. Moreover, the method provides the ability to proportionately quantify the relative amounts of a variant type to wild type. The addition of appropriate controls may provide additional quantitative information. The distribution and extent of DNA methylation within the nucleic acid target can also be determined using the present method. Other applications and uses will, of course, become apparent to one of ordinary skill in the art.

The present method can detect the presence of any type of genetic variation and the invention is not limited in this regard. Types of genetic variations particularly suited for detection using the present method include, without limitation, single nucleotide polymorphisms, clusters of polymorphisms, genetic disorders, allelic variations, exon sequences, intron sequences, and genetic subtypes. For example, the cytochrome P450 CYP2D6 subtype is known to have four different SNPs, one at each of four different exons within the cytochrome P450 CYP2D6 subtype. These variations of the cytochrome P450 CYP2D6 subtype are responsible for differential rates of drug metabolism. The present method can detect which variant of the cytochrome P450 CYP2D6 subtype occurs in an individual thereby assisting in the determination of how well the individual will metabolize a drug.

The nucleic acid analyte can be obtained from nearly any source. For example, the nucleic acid analyte can be obtained from a gene belonging to a patient or can be taken from a viral, bacterial, or fungal organism. Other sources will be evident to one of ordinary skill in the art.

It is to be understood that while the invention has been described in conjunction with the preferred specific embodiments thereof, that the description above as well as the examples which follow are intended to illustrate and not limit the scope of the invention. Other aspects, advantages and modifications within the scope of the invention will be apparent to those skilled in the art to which the invention pertains.

All patents, patent applications, and publications mentioned herein, both *supra* and *infra*, are hereby incorporated by reference.

### EXPERIMENTAL PROCEDURES

The following experimental procedures are set forth to provide those of ordinary skill in the art with a complete disclosure and description of how to make and use the hybridization probes and primers disclosed and claimed herein, and how to perform the methods using same; the examples are not intended to limit the scope of what the inventors regard as their invention. Efforts have been made to ensure accuracy with respect to numbers (e.g., amounts, temperature, etc.) but some errors and deviations should be accounted for. Unless indicated otherwise, parts are parts by weight, temperature is in degrees Celsius (°C), and pressure is at or near atmospheric pressure at sea level.

In the procedures set forth below and throughout this specification, the abbreviations employed have their generally accepted meanings, as follows:

| | |
|---|---|
| C | Celsius (or Centigrade) |
| mM | millimolar |
| µM | micromolar |
| pmol | picomole (10⁻¹² mole) |
| mg | milligram |
| µg | microgram |
| mL | milliliter |
| µL | microliter |
| µm | micrometer |
| Tm | melting temperature |
| U | units |

To carryout the procedures set forth below, a variety of software was used for primer design and Tm prediction, including Primer3, GCG®, VNTI, Primer Express®, and Hybsimulator. Hybsimulator was found to be the preferred software for Tm prediction.

### EXAMPLE

### DETECTION OF WILD TYPE OR MUTANT ALLELES FOR EXONS 1, 2, 6, AND 9 OF CYTOCHROME P450 CPY2D6

### PRIMER SEQUENCES FOR CYP2D6 MULTIPLEX SNP ASSAY:

As the first step in the detection of wild-type and mutant alleles for exons 1, 2, 6, and 9 of cytochrome P450 CPY2D6, the following forward and reverse primers were prepared:

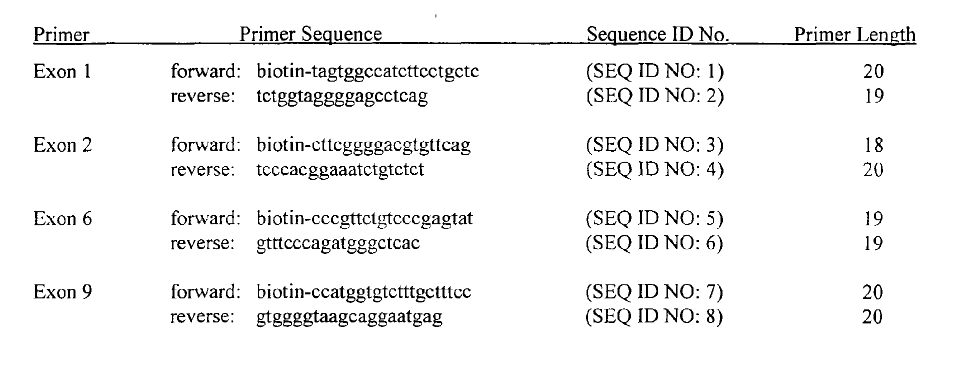

### EXTRACTION OF GENOMIC DNA:

As the second step in the detection of wild-type and mutant alleles for exons 1, 2, 6, and 9 of cytochrome P450 CPY2D6, human genomic DNA was extracted from 200 µL of EDTAtreated whole blood using QIAamp® DNA Blood Mini Kit as described by the manufacturer (Qiagen).

### DESIGN OF DIFFERENTIAL HYBRIDIZATION PROBES FOR CYP2D6 MULTIPLEX SNP ASSAY:

As the third step in the detection of wild type or mutant alleles for exons 1, 2, 6, and 9 of cytochrome P450 CPY2D6, allele specific hybridization probes were designed for use in a multiplexing mode. The presence or absence of a SNP in each of exons 1, 2, 6, and 9 was determined using allele specific hybridization probes comprising a sequence complementary to either the wild type or mutant allele, and further comprising unique capture sequences that are complementary to sequences attached to Luminex™ beads having unique color codes; the color detected indicating the identity of the allele specific sequence, and thus the presence of the wild type or mutant sequence in the target nucleotide sequence. The capture sequences were designed to minimize the potential non-specific binding to genomic DNA sequences that may be present in the amplified sequences by incorporating iso-cytosine and iso-guanine into the capture probes and complementary sequences attached to the Luminex™ beads.

The sequences shown below were designed and tested for their ability to discriminate between wild type and mutant cytochrome P450 genotypes in a sample of amplified genomic DNA, using the primers shown above to amplify the sequences of interest. The Tm for melting of each allele specific oligonucleotide sequence - target nucleotide sequence duplex is shown, relative to wild type sequences. Thus, as shown below, the allele specific hybridization probes designed to hybridize with wild type amplicon sequences are at least 10°C more stable (melting at least 10°C higher) than those allele specific hybridization probes designed to hybridize with mutant amplicon sequences. These melting temperatures are consistent with the predicted difference in melting temperatures for one base pair mismatched sequences of about 10°C. Similarly, the allele specific hybridization probe for mutant amplicon sequences will be more stable by at least 10°C when the mutant amplicon sequences are present. 
Exon 1 C  5'-JTATJCGCJCTGFTATJCCG  CCTGGTGGGTAGC  FGGCJFCTJGACJATFFTATCT
(SEQ ID NO: 9)                    Tm = 47.7 °C
 
Exon 1 T 5'-JTATJCGCJCTGFTATJCCG CCTGGTGAGTAGC AAGJTGGFAAJJAFCJTGCCT
(SEQ ID NO: 10) Tm = 36.3 °C
 
Exon 2 C  5'-JTATJCGCJCTGFTATJCCG  ATCTGGGTGATGGG  JAGGFACJTCJGATGFATFGCT
(SEQ ID NO: 11)                    Tm = 47.9 °C
 
Exon 2 T  5'-JTATJCGCJCTGFTATJCCG  ATCTGGATGATGGG  JATFGGFAACGFACFCTJGTGT
(SEQ ID NO: 12)                    Tm = 37.2 °C
 
Exon 6 C 5'-JTATJCGCJCTGFTATJCCG CTATGCGCAGGTTC TGCAFGFTGJAFAGAJCCAAFGT
(SEQ ID NO: 13) Tm = 48.4 °C
 
Exon 6 T 5'-JTATJCGCJCTGFTATJCCG CTATGCACAGGTTC GAJGATFCAFGGAJCCFTTJGAGTT
(SEQ ID NO: 14) Tm = 38.7 °C
 
Exon 9 G 5'-JTATJCGCJCTGFTATJCCG GATGGGCTCACCA AJCACTFGAJTGFGATJTAFCGJT
(SEQ ID NO: 15) Tm = 46.9 °C
 
Exon 9 C 5'-JTATJCGCJCTGFTATJCCG GATGGGGTCACCA CGGCFACJCTCTFAGFGAAJTGTT
(SEQ ID NO: 16) Tm = 26.5 °C
 Where J = iso-guanine, and F = iso-cytosine.

### SINGLEPLEX AND MULTIPLEX POLYMERASE CHAIN REACTIONS:

The fourth step in the detection of wild type or mutant alleles for exons 1, 2, 6, and 9 of cytochrome P450 CPY2D6, is the carrying out of a singleplex or multiplex polymerase chain reaction. In the present example, the singleplex or multiplex PCR was carried out using 2 µL (50-70 ng) isolated genomic DNA. Twenty-five microliter reaction volumes were prepared containing 1x Titanium Taq PCR buffer; 2.5 mM each dNTP's (dATP, dCTP, dGTP, and dTTP); 0.2µM each forward (biotinylated) and reverse primers; and 2.5 U Titanium Taq DNA polymerase. A PE 9600 thermocycler was used. The thermal cycling conditions were 94°C for 2 min followed by 30 cycles of 95°C for 30 see, 68°C for 1 minute, followed by a final extension of 5 min at 68°C.

### CYTOCHROME P450 SNP ASSAY:

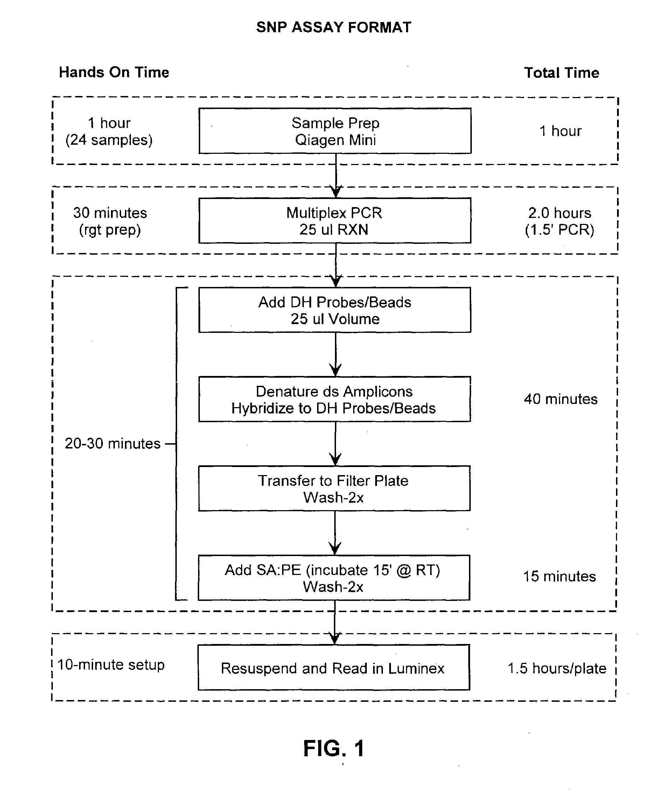

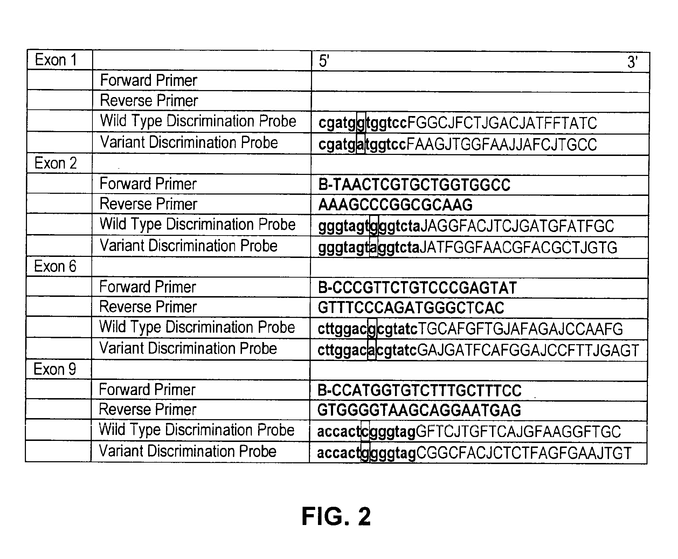

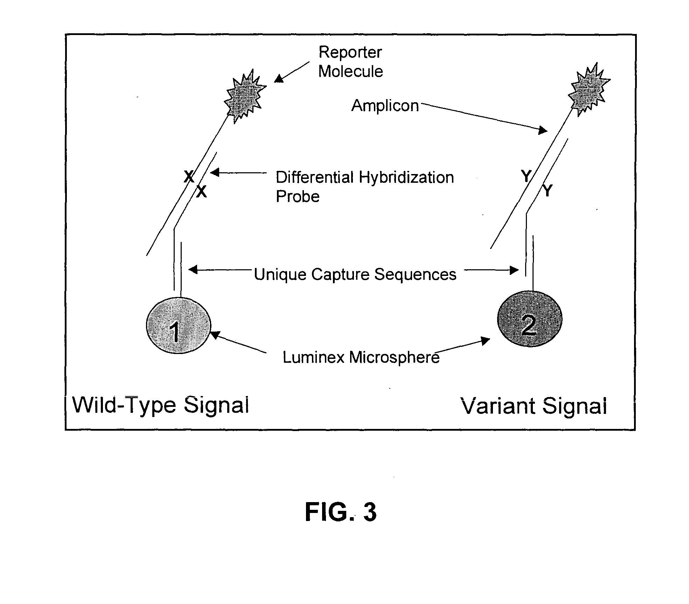

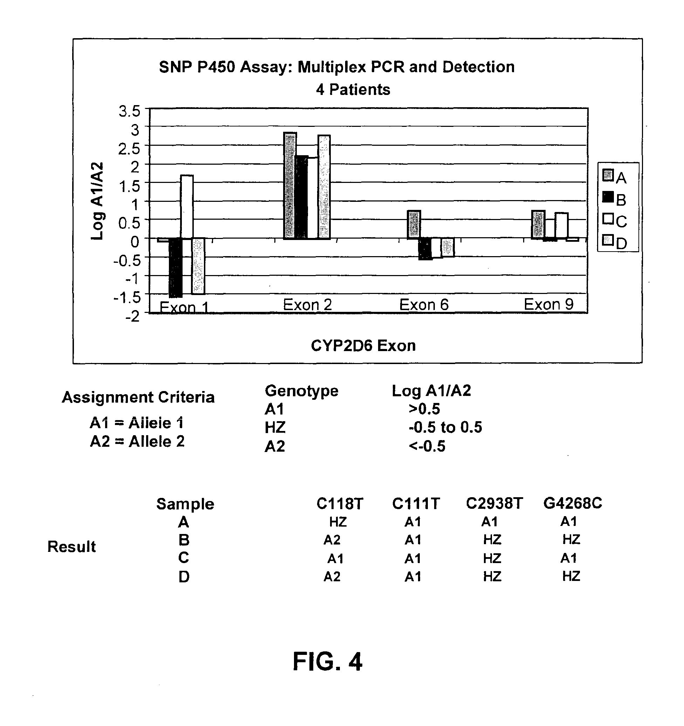

The final step in the detection of wild type or mutant alleles for exons 1, 2, 6, and 9 of cytochrome P450 CPY2D6, is the SNP Assay, shown in FIG. 1 and carried out as follows. After the PCR reaction was complete, 25 µL of multiplex working reagent was added to the individual wells containing the multiplex PCR products. The plate was sealed with mylar and the incubation was continued in the PE 9600 thermocycler. The multiplex working reagent contained 0.1 pmol each allele specific hybridization probe (ASH) for the four SNP regions and 2000 each individual Luminex™ microspheres per 25 µL of 50 mM Hepes containing 500 mM LiCl, 1 % LDS, 1% BSA, 10 mM MgCl₂, 0.01 mM ZnCl₂ and 0.5% sodium azide and Proclin-300 as a preservative (HIV 3.0 Label Diluent, Bayer Diagnostics), having a final pH of 7.5. The PCR plate with the multiplex ASH working reagent was incubated for 10 minutes at 95°C to dissociate double stranded DNA followed by a 30 minute incubation at 50°C to achieve allele specific hybridization. The plate was removed and 100 µL of wash buffer was added to each well (HIV 3.0 Wash A, Bayer Diagnostics). The contents of the wells were transferred to a 96 well pre-wetted filter plate (MultiScreen®-BV 1.2 µm, Millipore, Bedford MA). The wash buffer was pulled through with gentle vacuum and a 200 µL wash was repeated. The microspheres were resuspended in a 50 µL streptavidin phycoerythrin, (0.05µg/50µL) TTL buffer (50 mM Tris, 400 mM LiCl, 0.1% Tween-20, pH 8.0) mixture. The plate was then wrapped in aluminum foil and incubated for 15 minutes at 25°C with mild shaking (Titer Plate shaker, Labline Instruments). The previous wash step was repeated and the microspheres were resuspended in 80 µL of TTL buffer and read on the Luminex™ 100 in which the presence of phycoerythrin (and hence the original forward primer) and the microspheres specific for each particular ASH were detected. FIG. 2 shows the capture region for the wild type and variant discrimination probes for each of exons 1, 2, 6, and 9 of the cytochrome P450 CPY2D6 gene; FIG. 3 shows how the SNP assay results in the different color Luminex™ beads; and FIG. 4 shows the results of the SNP P450 Assay for four individual patients.

## Claims

1. A method for detecting the presence or absence of a genetic variation at a polymorphic site in a nucleic acid analyte in a sample, comprising:
(a) preparing labeled amplicons from the nucleic acid analyte contained in the sample;
(b) contacting, under hybridization conditions, the labeled amplicons with a plurality of first and second differential hybridization probes, wherein wild type complexes are formed between each first differential hybridization probe and a single labeled amplicon having a wild type sequence at the polymorphic site, and variant complexes are formed between each second differential hybridization probe and a single labeled amplicon having a variant sequence at the polymorphic site;
(c) contacting, under hybridization conditions, any wild type complexes and variant type complexes formed in step (b) with a plurality of first and second capture probes, wherein a captured wild type complex having a first detectable signal is formed between each wild type complex and a single first capture probe, and a captured variant complex having a second detectable signal is formed between each variant sequence and a single second capture probe;
(d) detecting and counting any captured wild type complexes and captured variant complexes formed in step (c); and
(e) determining the presence or absence of the genetic variation by comparing the relative amounts of the captured wild type and captured variant complexes detected in step (d), wherein a greater amount of captured wild type complexes is indicative of the absence of the genetic variation and a greater amount of captured variant complexes is indicative of the presence of the genetic variation.

2. The method of claim 1, wherein each first differential hybridization probe is comprised of a first capture sequence portion and a region that is complementary to the polymorphic site corresponding to the wild type sequence, and wherein each second differential hybridization probe is comprised of a second capture sequence portion and a region that is complementary to the polymorphic site corresponding to the variant sequence.

3. The method of claim 1 or 2, wherein each of the first and second differential hybridization probes has a spacer portion.

4. The method of claim 3, wherein each spacer portion of the first and second differential hybridization probes is located between the capture sequence portion and the region that is complementary to the polymorphic site.

5. The method of claim 2, wherein each first capture probe is (i) comprised of a region that is complementary to the first capture sequence portion and (ii) attached to a single solid substrate that provides the first detectable signal, and each second capture probe is (i) comprised of a region that is complementary to the second capture sequence portion and (ii) attached to a single separate solid substrate that provides the second detectable signal.

6. The method of any of the preceding claims, wherein the first and second detectable signals are provided by moieties which are fluorescers, chemiluminescers, dyes, biotin, haptens, enzymes, enzyme substrates, enzyme cofactors, enzyme inhibitors, enzyme subunits, metal ions, electron-dense reagents or radioactive isotopes.

7. The method of claim 6, wherein the first and second detectable signals are provided by one or more fluorescent dyes.

8. The method of claim 7, wherein the first and second detectable signals are provided by two fluorescent dyes.

9. The method of claim 8, wherein the first detectable signal is provided by a ratio of the two different fluorescent dyes and the second detectable signal is produced by a different ratio of the two different fluorescent dyes.

10. The method of any of the preceding claims, wherein step (c) is performed by running the captured wild type and variant complexes through a flow cytometer designed to detect and count both the detectable signals and the label of the labeled amplicon.

11. The method of claim 10, wherein detection of the label of the labeled amplicon and the first detectable signal is indicative of a captured wild type complex and wherein detection of the label of the labeled amplicon and the second detectable signal is indicative of a captured variant complex.

12. The method of any of the preceding claims, wherein the labeled amplicons are PCR products produced via PCR synthesis with labeled primers.

13. The method of claim 12, wherein the labeled primers are biotinylated primers.

14. The method of any of the preceding claims, wherein the labeled amplicons are directly or indirectly labeled with a moiety which is a fluorescer, chemiluminescer, dye, biotin, hapten, enzyme, enzyme substrate, enzyme cofactor, enzyme inhibitor, enzyme subunit, metal ion, electron-dense reagent or radioactive isotope.

15. The method of any of the preceding claims, wherein the sample is obtained from a patient.

16. The method of any of the preceding claims, wherein the genetic variation is a single nucleotide polymorphism or a cluster of polymorphisms, or is associated with a genetic disorder, allelic variation, exon sequence, an intron sequence or a genetic subtype.

17. The method of any of the preceding claims, wherein the nucleic acid analyte is obtained from a gene belonging to a patient or wherein the nucleic acid analyte belongs to the gene of an infectious agent which is a viral, bacterial or fungal organism.

18. The method of any of the preceding claims, in multiplex form wherein a plurality of genetic variations is detected through use of a plurality of different differential hybridization probes and capture probes.

19. Use of a method of any of the preceding claims to predict phenotype.

20. An assay kit for detecting the presence or absence of a genetic variation at a polymorphic site in a nucleic acid analyte in a sample, comprising:
(a) a plurality of first differential hybridization probes each comprised of a first capture sequence portion and a region that is complementary to the polymorphic site corresponding to a wild type sequence;
(b) a plurality of second differential hybridization probes each comprised of a second capture sequence portion different from the first capture sequence portion and a region that is complementary to the polymorphic site corresponding to a variation sequence;
(c) a plurality of first solid substrates each (i) comprised of an attached first capture probe complementary to the first capture region and (ii) having a first detectable signal; and
(d) a plurality of second solid substrates each (i) comprised of an attached second capture probe complementary to the second capture region and (ii) having a second detectable signal.

21. The assay kit of claim 20, wherein each first and second solid substrate is a bead.

22. The assay kit of claim 20 or 21, wherein the first and second detectable signals are provided by one or more fluorescent dyes.

23. The assay kit of claim 22, wherein the first and second detectable signals are provided by two dyes.

24. The assay kit of claim 23, wherein the first detectable signal is provided by a ratio of the two different dyes and the second detectable signal is produced by a different ratio of the two different dyes.

25. The assay kit of any of claims 20 to 24, further comprising a polymerase for amplifying nucleic acids and/or primers for carrying out PCR.

26. The assay kit of claim 25, wherein the primers are labeled.

27. The assay kit of claim 26, wherein the labeled primers are biotinylated primers.

28. The assay kit of any of claims 20 to 27, further comprising instructions for carrying out an assay for detecting the presence or absence of a genetic variation.
